# EUROPEAN PATENT SPECIFICATION

(11) **EP 0 606 853 B1**
(45) Date of publication and mention of the grant of the patent: **19.11.1997**
(21) Application number: 94100167.9
(22) Date of filing: 07.01.1994
(51) Int. Cl.: C07H 1/06, C07H 11/04, A61K 31/70

(54) **New process for obtaining octahydro trisodium salt of fructose 1,6-diphosphate (FdPNa3H*8H20) in a crystalline form**
Neues Verfahren zur Gewinnung des Oktahydro-Trinatriumsalzes von Fructose-1,6-diphosphat (FdPNa3H*8H2O) in einer kristallinen Form
Procédé nouveau de préparation du sel octahydro-trisodique de fructose-1,6-diphosphate (FdPNa3H*8H2O) en forme cristalline

(30) Priority: 13.01.1993 IT MI930029
(43) Date of publication of application: 20.07.1994
(73) Proprietor: BIOMEDICA FOSCAMA INDUSTRIA CHIMICO-FARMACEUTICA S.P.A., I-00131 Roma (IT)
(72) Inventor: CECCARELLI, Stefano, 03100 FROSINONE (IT); MAGNANTE, Francesco, 00034 COLLEFERRO (RM) (IT); ZANARELLA, Sergio, 00010 MENTANA (RM) (IT)
(74) Representative: La Ciura, Salvatore

(56) References cited:
- DE-A- 2 061 731

## Description

The present invention relates to a new process for obtaining octahydro trisodium salt of fructose 1,6-diphosphate in a crystalline form by using aqueous solution of FdP.

Fructose 1,6-diphosphate has been isolated by Young for the first time in 1909, and at present it is widely used as drug for the treatment of many pathological conditions.

It is usually produced as sodium salt in an amorphous form, and as such it is extremely hygroscopic and deliquescent in the air. The difficulties arised during the storage and the transport of the product, as well as in the preparation of pharmaceutical compositions, are to be ascribed to such properties.

In DE 2,061,731 the crystalline octahydro form of trisodium salt of fructose 1,6-diphosphate (FdPNa3H*8H2O), obtained by crystallization from a hydroalcoholic solution of FdP, is described. Such a form, compared to the amorphous one, is not hygroscopic and it is remarkably more stable. Although effective, the process as above mentioned involves the consumption of great amounts of ethanol, the recovery of which from the mother liquors is difficult because of the physicochemical properties of hydroalcoholic mixtures. Moreover this process involves the use of remarkable volumes of hydroalcoholic solution for unity of products, thus causing the lost of one part of the product (from 4 to 9%), that remains dissolved in the mother liquors. Moreover, the special fiscal status to which the alcohols are subjected inveitably raises the costs for industrial production.

By the present invention it was surprisingly found that the crystallization of octahydro trisodium salt of fructose 1,6-diphosphate occurs even from water/acetone mixtures, with excellent results as concerns yields and quality of the obtained product. Such a result is particularly unexpected if it is considered that the use of alcohols homologous of ethanol (methanol, n-propanol, isopropanol) leads to unsatisfied results, as already reported in the mentioned document DE 2,061,731.

Moreover the process described in the present invention has the remarkable economical advantage of an easy and almost quantitative recovery of acetone by a simple distillation of the process mother liquors.

Thus this solvent can be reused for producing a further batch of product without a remarkable deterioration of its quality and without any lowering of the yield.

Moreover, the process of the present invention remarkably reduces the product amount that is lost in the mother liquors: said amount is comprised from 0,2 to 2% of the product initially used. Finally, a further advantage of the present invention is that it allows the use of smaller volumes per unit of obtained product:
this can be obtained by the fact that the process herein described applies to starting from solutions having up to 20% (w/v) of fructose 1,6-diphosphate.

The process described in the present invention provides raising the pH value of, a fructose 1,6-diphosphate aqueous solution by a suitable base such as NaOH at a temperature from 0 to 15°C. as a first step: the pH to be obtained must be comprised between 5.5 and 6.3, preferably between 5.8 and 6.0; the solution thus obtained, that will to have a content of FdP comprised between from 70 and 200 mg/ml, is mixed with a first aliquot of acetone as to reach a lasting clouding of the mixture; said mixture is then poured under stirring into a further amount of acetone suitably cooled.

The total amount of acetone is from two to three times the starting aqueous solution.

After stirring, for a suitable time the suspension is kept at rest at low temperature up to the completion of crystallization. In a preferred embodiment of the invention the temperature of the mixture during the different steps of the process up to the end of crystallization does not go over 10°C. The solid thus obtained is filtered, washed by acetone and dried up to a constant weight. The drying is made at temperatures lower than 30°C and without the aid of dehydrating agents in order to avoid the loss of crystallization water.

The following example particularly describes the invention without limiting it.

### EXAMPLE

A solution of FdP sodium salt (1.4 l, 111 mg/ml in FdPH4, pH 5.3) is leaded adjusted to pH=5.85 by about 120 ml of 2N NaOH keeping the temperature lower than 10°C.

500 ml of acetone cooled at 5°C is then added, with the mixture again kept at a temperature lower than 10°C, thus obtaining the lasting clouding of the solution. The mixture is then poured (with a flow of about 70 ml/min) into 3.6 l of acetone cooled at 2°C and kept under vigorous stirring: during the addition the temperature of the mixture does not go over 3°C. After a 12 hours stirring, the solution is kept at rest for further 10 hours at 2°C. The suspension is thus filtered under reduced pressure and the white solid is washed by acetone (2 x 80 ml). Then it is dried under 1000-2000 Pa (10-20 mbar) at room temperature in the presence of activated carbon up to a constant weight, thus obtaining 228.8 g of product (chemical yield 90%). The mother liquors (total volume 5.2 l) are clear and colourless, and can be distilled again under atmospheric pressure for the recovery of acetone (3.3-3.4 l, Tdist.=58-62°C, assay 96-97%).

The product analysis shows values in accordance with formula C6H11012P2Na3*8H2O (M.W. 550.18):

| | Found % | Calculated % |
|---|---|---|
| FdPH4 | 61.2 | 61.8 |
| Na | 12.9 | 12.5 |
| H2O | 25.4 | 26.2 |
| P inorg. | 0.13 | |
| Fructose-6P | 0.36 | |
| Glucose-6P | 0.13 | |

## Claims

1. A process for producing octahydro trisodium salt of fructose 1,6-diphosphate in a crystalline form characterized in that an aqueous solution of D-fructose 1,6-diphosphate is adjusted to a pH from 5.5 to 6.3 by a suitable base at a temperature from 0 to 15°C, thus obtaining a concentration of fructose 1,6-diphosphate varying from 70 to 200 mg/ml; this solution is mixed, in two consecutive steps with a total volume of acetone equal to 2 up to 3 times the volume of the starting aqueous solution; after crystallization and filtration of the reaction mixture, the desired product is isolated and then dried.

2. A process according to claim 1, characterized in that the aqueous solution of D-fructose 1,6-diphosphate is adjusted to pH from 5.8 to 6.0 by a suitable base.

3. A process according to anyone of claims 1 and 2, characterized by the fact that the used base is NaOH.

4. A process according to anyone of claims from 1 to 3, characterized by the fact that, after adding the base, the fructose 1,6-diphosphate has a concentration of about 200 mg/ml in aqueous solution.

5. A process according to anyone of claims from 1 to 4, characterized by the fact that an aliquot of the total volume of acetone is added up to obtain a lasting clouding of the reaction mixture, which is then added to the remaining volume of acetone and stirred until the end of crystallization.

6. A process according to anyone of claims from 1 to 5, characterized by the fact that, during its different steps up to the end of crystallization, the mixture temperature does not go over 10°C.

## Patentansprüche

1. Verfahren zur Herstellung eines Octahydro-Trinatriumsalzes von Fructose-1,6-diphosphat in kristalliner Form, dadurch gekennzeichnet, daß eine wässerige Lösung von D-Fructose-1,6-diphosphat auf einen pH-Wert von 5,5 bis 6,3 mit einer geeigneten Base bei einer Temperatur von 0 bis 15°C eingestellt wird, so daß eine Konzentration an Fructose-1,6-diphosphat, die von 70 bis 200 mg/ml schwankt, erhalten wird; diese Lösung in zwei aufeinanderfolgenden Schritten mit einem Gesamtvolumen an Aceton gleich dem 2- bis zu 3-fachen Volumen der wässerigen Ausgangslösung vermischt wird; nach Kristallisation und Filtration des Reaktionsgemisches das gewünschte Produkt abgetrennt und anschließend getrocknet wird.

2. Verfahren nach Anspruch 1, dadurch gekennzeichnet, daß die wässerige Lösung von D-Fructose-1,6-diphosphat mit einer geeigneten Base auf pH 5,8 bis 6,0 eingestellt wird.

3. Verfahren nach einem der Ansprüche 1 und 2, dadurch gekennzeichnet, daß die verwendete Base NaOH ist.

4. Verfahren nach einem der Ansprüche 1 bis 3, dadurch gekennzeichnet, daß nach der Zugabe der Base das Fructose-1,6-diphosphat eine Konzentration von etwa 200 mg/ml in wässeriger Lösung aufweist.

5. Verfahren nach einem der Ansprüche 1 bis 4, dadurch gekennzeichnet, daß eine aliquote Menge des Gesamtvolumens an Aceton bis zur anhaltenden Trübung des Reaktionsgemisches zugegeben wird, das anschließend zu dem verbleibenden Volumen an Aceton gegeben wird und daß bis zum Ende der Kristallisation gerührt wird.

6. Verfahren nach einem der Ansprüche 1 bis 5, dadurch gekennzeichnet, daß während seiner verschiedenen Schritte die Temperatur des Gemisches bis zum Ende der Kristallisation 10°C nicht überschreitet.

## Revendications

1. Procédé de production du sel octahydro-trisodique de fructose 1, 6-diphosphate en forme cristalline, caractérisé en ce qu'une solution aqueuse de D-fructose 1, 6-diphosphate est ajustée à pH allant de 5,5 à 6,3 au moyen d'une base convenable à une température allant de 0 à 15°C, obtenant ainsi une concentration en fructose 1, 6-diphosphate variant de 70 à 200 mg/ml; en ce que cette solution est mélangée, au cours de deux étapes successives, avec un volume total d'acétone égal à 2 jusqu'à 3 fois le volume de la solution aqueuse de départ; en ce qu'après cristallisation et filtration du mélange réactionnel, le produit recherché est isolé et ensuite séché.

2. Procédé selon la revendication 1, caractérisé en ce que la solution aqueuse de D-fructose 1, 6-diphosphate est ajustée à pH allant de 5,8 à 6,0 au moyen d'une base convenable.

3. Procédé selon l'une quelconque des revendications 1 et 2, caractérisé en ce que la base utilisée est NaOH.

4. Procédé selon l'une quelconque des revendications 1 à 3 caractérisé en ce que, après adjonction de la base, la concentration de fructose 1, 6-diphosphate en solution aqueuse est d'environ 200 mg/ml.

5. Procédé selon l'une quelconque des revendications 1 à 4, caractérisé en ce qu'une fraction aliquote du volume total d'acétone est ajoutée jusqu'à ce que le mélange réactionnel adopte un aspect trouble stable, qui est ensuite ajouté au volume d'acétone résiduel et agité jusqu'en fin de cristallisation.

6. Procédé selon l'une quelconque des revendications 1 à 5, caractérisé en ce que, durant les différentes étapes de son exécution et jusqu'en fin de cristallisation, la température du mélange ne dépasse pas 10°C.
